# EUROPEAN PATENT APPLICATION

(11) **EP 4 772 129 A1**
(43) Date of publication of application: **08.07.2026**
(21) Application number: 24860055.3
(22) Date of filing: 24.05.2024
(51) Int. Cl.: A61B 34/00, A61B 17/00, A61B 90/00, A61B 34/20

(54) **MAGNETIC DRIVING SYSTEM**

(30) Priority: 28.08.2023 KR 20230112494
(71) Applicant: Daegu Gyeongbuk Institute Of Science and Technology, Daegu 42988 (KR)
(72) Inventor: CHOI, Hong Soo, Daegu 42951 (KR); LEE, Hak Joon, Daegu 43014 (KR); LATIFI, Gharamaleki Nader, Daegu 42988 (KR); KIM, Jin Young, Daegu 43016 (KR)
(74) Representative: dompatent
(86) International application number: PCT/KR2024/007087
(87) International publication number: WO 2025/048129

(57) **Abstract**

A magnetic driving system according to the present disclosure comprises: a bed; a first magnetic generator installed on one side of the bed; a second magnetic generator installed on the rear surface of the bed; an imaging device connected to the first and second magnetic generators; and a controller for controlling the first and second magnetic generators and the imaging device.

## Description

### [Technical Field]

The present invention relates to a magnetic driving system for use in medical devices.

### [Background Art]

Recently, medical equipment utilizing microrobots and electromagnetic devices has been used in medical settings. These microrobots are small, implantable within the human body, and require fine adjustments from outside the body. To achieve this, electromagnetic devices capable of fine adjustments have been utilized to drive microrobots and other devices in the field.

Conventional electromagnetic devices used in medical equipment utilize electromagnetic coils. However, these electromagnetic coils generate excessive heat, making them unsuitable for long-term use in medical equipment. In addition, it has been difficult to precisely control the direction of a magnetic field or the magnitude of magnetic force as required for medical devices requiring fine control.

In addition, the mechanical movement of electromagnetic coils, which operates in real time, significantly limits operators' access to patients. In addition, poor compatibility with medical imaging systems, such as X-ray systems, has resulted in severely limited angles available for image capturing.

Prior art document includes Japanese Application Publication No. 2021-522960 (published on September 2, 2021).

### [Disclosure]

### [Technical Problem]

An object of the present invention is to provide a magnetic driving device and a system using the same and provide a magnetic driving system capable of effectively controlling the direction and intensity of a magnetic field.

### [Technical Solution]

In one general aspect, a magnetic driving system includes: a bed; a first magnetic generator installed on a rear surface of the bed; a second magnetic generator installed on one side of the bed; an imaging device connected to the first and second magnetic generators; and a controller controlling the first and second magnetic generators and the imaging device.

In another general aspect, a magnetic driving system includes: a bed; a first magnetic generator installed on a rear surface of the bed; a second magnetic generator installed on both sides of the bed; an imaging device connected to the first and second magnetic generators; and a controller controlling the first and second magnetic generators and the imaging device.

The first magnetic generator may include a first magnetic coil around which a coil is wound and having a first coil tip formed at one end thereof, wherein at least two of the first magnetic coils may be connected by a magnetic yoke at the other ends of the first magnetic coil.

At least two of the first magnetic generators may be arranged on the rear surface of the bed such that the first coil tips face each other.

The first coil tip may have a protrusion with an end facing the bed.

The first coil tip may have a protrusion facing the bed and is inclined at a predetermined angle toward the bed.

The first coil tip may have a bent end to face the bed.

The second magnetic generator may include a second magnetic coil around which a coil is wound and having second coil tips formed at both ends thereof.

The second coil tip may have a protrusion with both ends facing the bed.

The second coil tip may be bent at both ends to face the bed.

The second magnetic generator may be provided in plurality.

At least two first magnetic generators may be connected to each other by extending the magnetic yoke.

The first magnetic generator and the second magnetic generator may be connected to each other by a magnetic yoke.

A cross-section of the first magnetic coil may have a circular or square shape.

A cross-section of the second magnetic coil may have a circular or square shape.

### [Advantageous Effects]

The magnetic driving system according to the present invention may effectively control the direction and intensity of a magnetic field in a magnetic generator by enabling a magnetic coil tip provided in a magnetic coil to be oriented toward a bed in which a patient is lying.

In addition, by eliminating driving elements that limit space utilization, it is possible to perform safe procedures for both a practitioner using medical equipment and a patient.

### [BRIEF DESCRIPTION OF THE DRAWINGS]

FIG. 1 is a conceptual diagram of a magnetic driving system according to the present invention.
FIG. 2 is a diagram separately illustrating first and second magnetic generators according to an embodiment of the magnetic driving system according to the present invention.
FIG. 3 is a side view of a bed and the first and second magnetic generators in FIG. 1.
FIG. 4 is a side view of a first magnetic generator of a magnetic driving system according to an embodiment of the present invention.
FIG. 5 is a perspective view of the first magnetic generator of a magnetic driving system according to another embodiment of the present invention.
FIG. 6 is a perspective view of the first magnetic generator of a magnetic driving system according to another embodiment of the present invention.
FIG. 7 is a perspective view of a second magnetic generator of a magnetic driving system according to another embodiment of the present invention.
FIGS. 8 through 10 are perspective views illustrating the second magnetic generator according to other embodiments of the present invention.
FIGS. 11 through 14 are diagrams illustrating simulation results of magnetic fields generated by a magnetic driving system according to the present invention.

### [Detailed Description of Main Elements]

100: Bed
200: First Magnetic Generator
210: First Magnetic Coil
211: Coil
212: First Coil Tip
2121: Protrusion
220: Magnetic Yoke
300: Second Magnetic Generator
310: Second Magnetic Coil
311: Coil
312: Second Coil Tip
3121: Protrusion
400: Imaging Device
500: Controller

### [Best Mode]

Hereinafter, specific embodiments of the present invention will be described in detail with reference to the drawings.

In addition, in describing the present invention, if a detailed description of a related known structure or function is determined to obscure the gist of the present invention, the detailed description will be omitted.

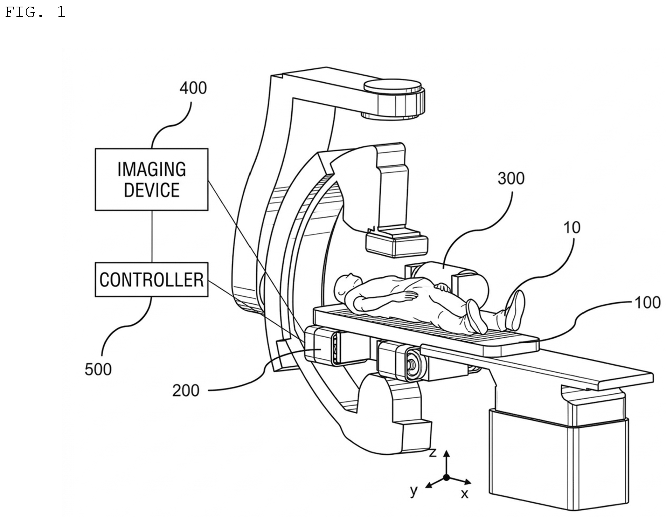

Referring to FIG. 1, a magnetic driving system according to the present invention may include a bed 100, a first magnetic generator 200, a second magnetic generator 300, an imaging device 400, and a controller 500.

The bed 100 is a bed on which a patient 10 lies or sits to receive treatment or examination with a magnetic robot or the like using the first magnetic generator 200 and the second magnetic generator 300.

The first and second magnetic generators 200 and 300 are installed on the bed 100. The first magnetic generator 200 may be installed on one side of the bed 100, and the second magnetic generator 300 may be installed on a rear surface of the bed 100. The arrangement of the first and second magnetic generators 200 and 300 is designed to avoid interference with the imaging device 400.

The imaging device 400 is connected to the first and second magnetic generators 200 and 300. The imaging device 400 is a device, such as a monitor or display, used to monitor a location of the magnetic robot and a lesion or a treatment process in real time. The imaging device 400 may also track the location of the magnetic robot in real time.

The controller 500 connects to the first and second magnetic generators 200 and 300 and the imaging device 400 and controls them individually. The controller 500 includes a computer device, etc. For example, the imaging device 400 and the controller 500 may be configured as a single device.

The imaging device 400 and the controller 500 may be general medical equipment used in hospitals, and there are no limitations as long as they may be connected to the first and second magnetic generators 200 and 300.

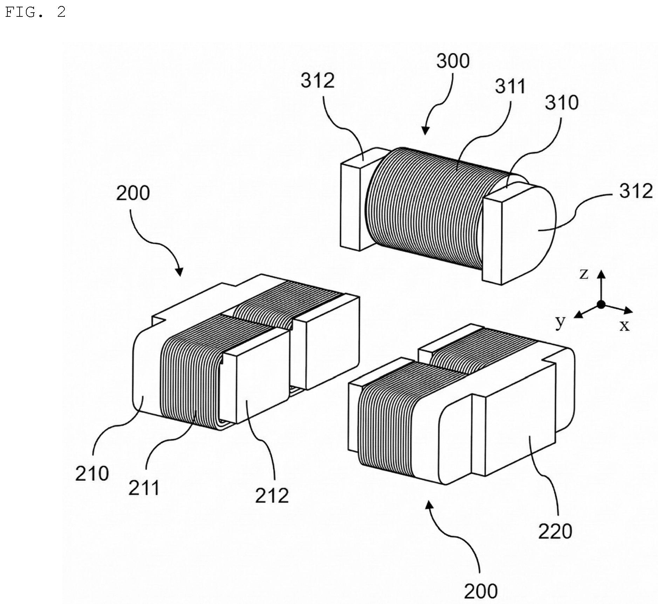

FIG. 2 illustrates only the first and second magnetic generators 200 and 300 with the bed 100 removed from FIG. 1.

Referring to FIG. 2 together, the first magnetic generator 200 according to an embodiment includes a first magnetic coil 210, which may include at least two first magnetic coils 210.

The first magnetic coil 210 is wound around a coil 211 to generate magnetic force by applying current. The first magnetic coil 210 has a first coil tip 212 formed at one end. At least two first magnetic coils 210 may be connected to a magnetic yoke 220 on the other side of the first magnetic coil 210.

In the first magnetic generator 200, each first magnetic coil 210 may be disposed such that the first coil tips 212 face in the same direction. At least two first magnetic coils 210 may be positioned on the same plane (the z-y plane as shown in FIG. 2).

The magnetic yoke 220 is a component that reinforces the magnetic field of the first magnetic coil 210 and is formed of the same material as the first magnetic coil 210. The magnetic yoke 220 may reinforce the magnetic field in the y-axis direction as shown in FIG. 2.

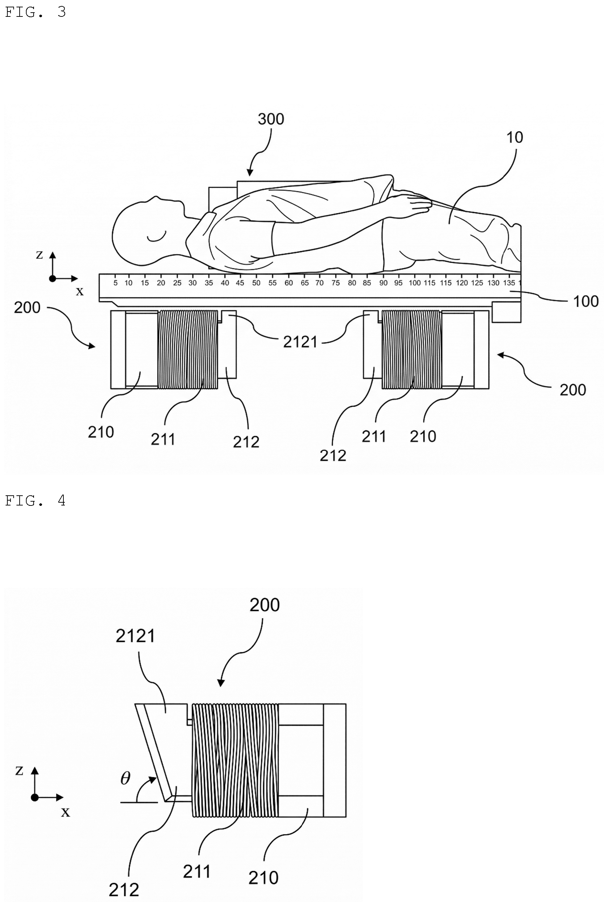

Referring to FIG. 3, at least two first magnetic generators 200 according to an embodiment may be positioned on the rear surface of the bed 100. One of the first magnetic generators 200 may be positioned above the rear surface of the bed 100 so as to be positioned over the head of the patient 10, and the other may be positioned below the rear surface of the bed 100 so as to be positioned over the lower body of the patient 10. In this case, the at least two first magnetic generators 200 may be positioned such that the first coil tips 212 thereof face each other.

In an embodiment, the first coil tip 212 may include a protrusion 2121 having an end facing the bed 100. The protrusion 2121 may protrude in a direction toward the bed 100 such that the first coil tip 212 points toward an affected area of the patient 10. Referring to FIG. 3, the protrusion 2121 may be oriented in the Z-axis direction.

Referring to FIG. 4, as another embodiment of the first coil tip 212, the first coil tip 212 may be provided with the protrusion 2121 oriented toward the bed 100 but may be inclined at a predetermined angle *θ* toward the bed 100. The angle *θ* is defined as an angle formed between the direction (the Z-axis direction in FIG. 4) of the bed 100 and a direction (the X-axis direction in FIG. 4) parallel to the bed 100. It is preferable that the angle *θ* be formed to be an acute angle.

In addition, in another embodiment, the first coil tip 212 may be formed with a bent end to face the bed 100.

Referring to FIG. 2, the second magnetic generator 300 includes a second magnetic coil 310 around which a coil is wound and having second coil tips 312 formed on both sides thereof.

The second magnetic generator 300 may be disposed on one side of the patient 10, as illustrated in FIGS. 1 and 3. The second magnetic generator 300 may be installed on one side of the bed 100 so as to be positioned on the side of the patient 10 lying on the bed 100.

Here, the second coil tip 312 of the second magnetic generator 300 may be formed at both ends of the second magnetic coil 310. By forming the second coil tip 312 at both ends of the second magnetic coil 310, the direction of the magnetic field may be directed toward the patient 10.

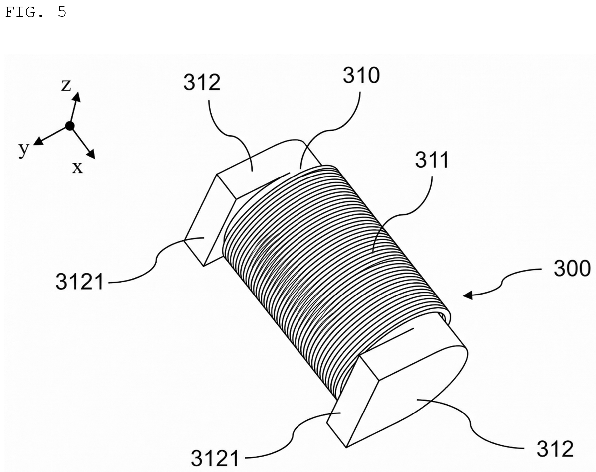

In an embodiment of the second magnetic generator 300, referring to FIG. 5, the second coil tips 312 may include the protrusions 3121 such that both ends thereof face the bed 100. By forming the protrusions 3121 at both ends of the second coil tips 312, the protrusions 3121 may be oriented toward the patient 10 lying on the bed 100. Referring to FIGS. 1 and 2 together, the protrusions 3121 may protrude in a direction toward the bed 100 such that the second coil tips 312 point toward an affected area of the patient 10. Based on FIG. 3, the protrusions 3121 may be oriented in the Y-axis direction.

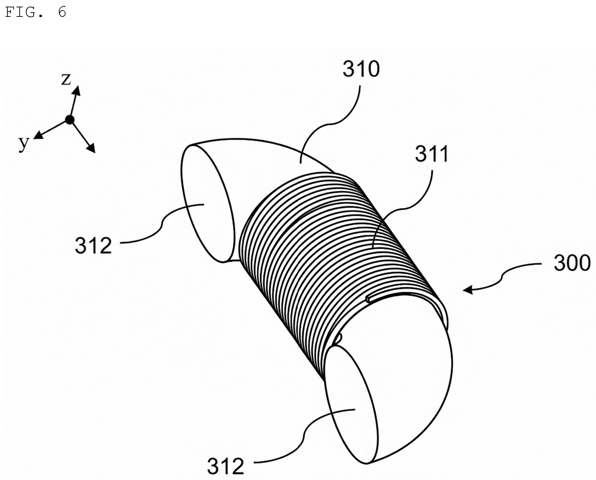

In another embodiment of the second magnetic generator 300, referring to FIG. 6, both ends of the second coil tips 312 may be bent to face the bed 100. Referring to FIG. 6, the second coil tips 312 may be formed to be bent toward the Y-axis direction. The entire surfaces of the second coil tips 312 are oriented toward the Y-axis direction

When the second magnetic generator 300 is installed on only one side of the bed 100, the strength of magnetic field may be strengthened in the X-axis direction. In addition, leaving the other side of the bed 100 empty may facilitate the operator's access to the patient.

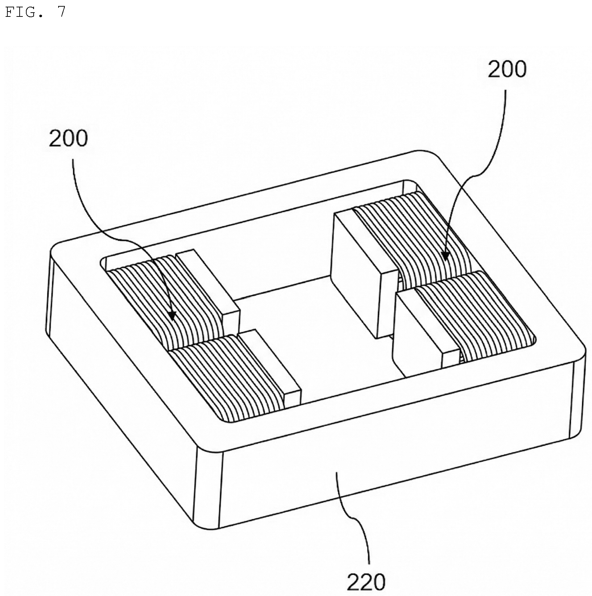

Referring to FIG. 7, at least two first magnetic generators 200 may be connected to each other by extending the magnetic yoke 220. At least two first magnetic generators 200 may be connected to a magnetic yoke 220 to generate a strong magnetic field.

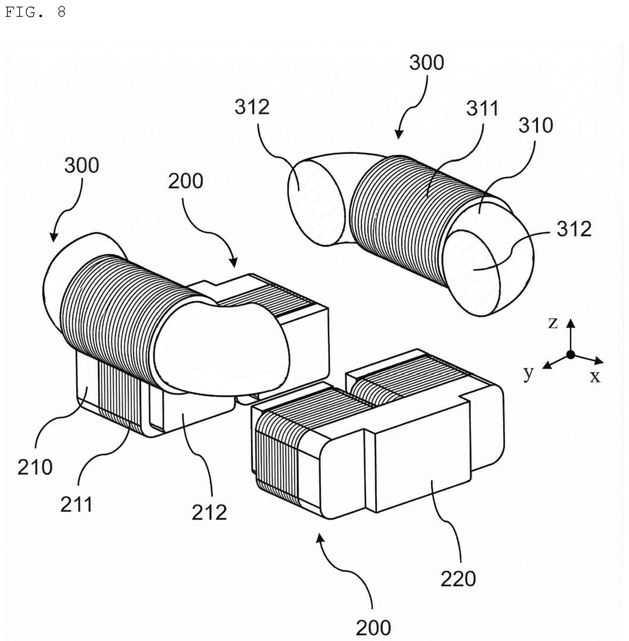

Referring to FIG. 8, the second magnetic generators 300 may be installed on both sides of the bed 100. At least two second magnetic generators 300 may be installed to face each other on both sides of the bed 100. In this case, the second coil tips 312 of different second magnetic generators 300 may be disposed to face each other.

When the second magnetic generators 300 are installed on both sides of the bed 100, the strength of the magnetic field in the X-axis and Y-axis directions may be strengthened. Such an arrangement may be necessary when a strong magnetic field is required.

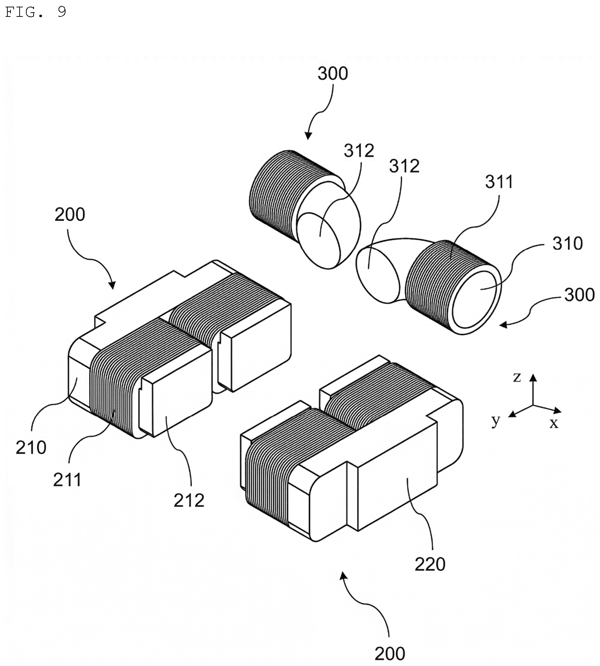

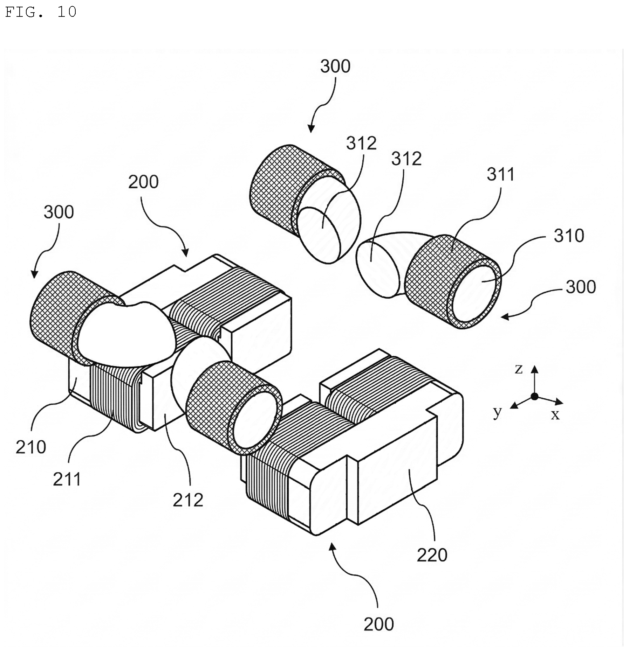

Referring to FIGS. 9 and 10, a plurality of second magnetic generators 300 may be provided. Even when the second magnetic generator 300 is installed on only one side of the bed 100, as illustrated in FIG. 9, a plurality of second magnetic generators 300 may be arranged. The same is applied to a case in which the second magnetic generators 300 are installed on both sides, as illustrated in FIG. 10.

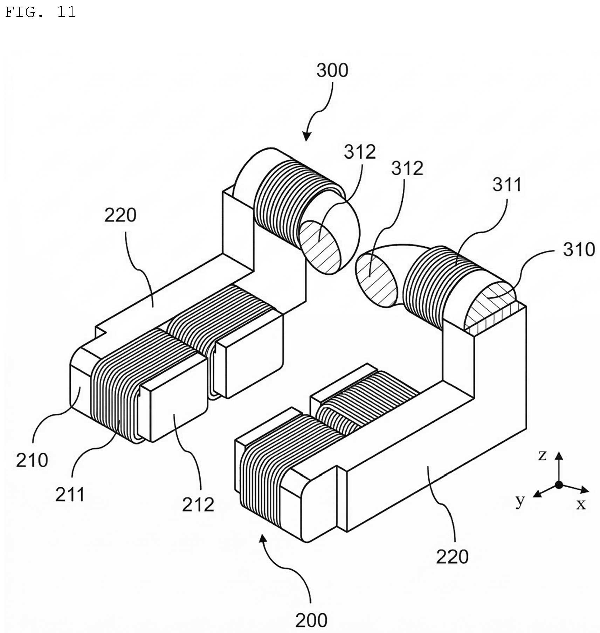

Referring to FIG. 11, the first magnetic generator 200 and the second magnetic generator 300 may be connected by a magnetic yoke 220.

The cross-sections of the first magnetic coil 210 and the second magnetic coil 310 in the first magnetic generator 200 and the second magnetic generator 300 may have circular or rectangular shapes.

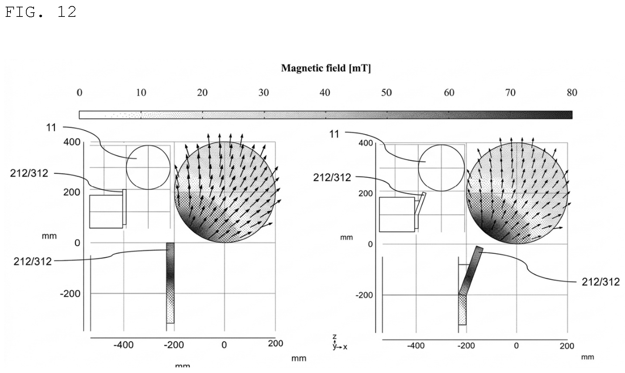

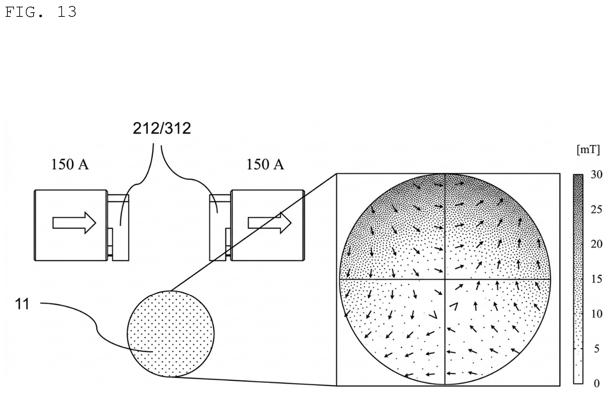

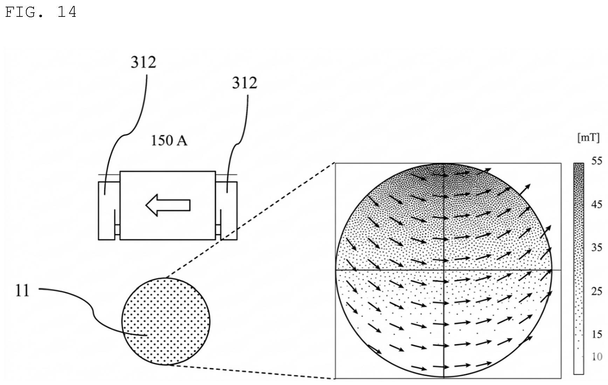

FIGS. 12 to 14 illustrate the strength of a magnetic field of the embodiments described above.

Referring to FIG. 8, it can be seen that the strength of a magnetic field may be varied depending on the direction pointed by the first magnetic coil tip 212 or the second magnetic coil tip 212 and 312. (Hereinafter, the description will be based on the first magnetic coil tip 212.) Reference numeral 11 is a schematic representation of the affected area of the patient. As can be seen in the graph on the right, when the first magnetic coil tip 212 is bent toward the affected area 11, a stronger magnetic field is generated toward the affected area 11.

FIG. 9 shows two first magnetic coil tips 212 configured to point toward the affected area 11. Also, by configuring the first magnetic coil tip 212 to point toward the affected area 11, a strong and uniform magnetic field may be generated in the affected area 11.

FIG. 10 shows a configuration in which the second magnetic coil tips 312 at both ends of the second magnetic generator 300 are directed toward the affected area 11. It can be seen that, by configuring the second magnetic coil tips 312 to point toward the affected area 11, a strong and uniform magnetic field may be generated in the affected area 11.

Although the magnetic driving device using a permanent magnet and the system using the same according to the embodiments of the present invention have been described in specific embodiments, these are merely examples, and the present invention is not limited thereto and should be construed as having the broadest scope in accordance with the basic idea disclosed in this specification. Those skilled in the art may implement embodiments not specified by combining or replacing the disclosed embodiments, but this also does not exceed the scope of the present invention. In addition, those skilled in the art may easily change or modify the disclosed embodiments based on this specification, and it is clear that such changes or modifications also fall within the scope of the present invention.

## Claims

1. A magnetic driving system comprising:
a bed;
a first magnetic generator installed on a rear surface of the bed;
a second magnetic generator installed on one side of the bed;
an imaging device connected to the first and second magnetic generators; and
a controller controlling the first and second magnetic generators and the imaging device.

2. A magnetic driving system comprising:
a bed;
a first magnetic generator installed on a rear surface of the bed;
a second magnetic generator installed on both sides of the bed;
an imaging device connected to the first and second magnetic generators; and
a controller controlling the first and second magnetic generators and the imaging device.

3. The magnetic driving system of claim 1, wherein
the first magnetic generator includes a first magnetic coil around which a coil is wound and having a first coil tip formed at one end thereof,
wherein at least two of the first magnetic coils are connected by a magnetic yoke at the other ends of the first magnetic coil.

4. The magnetic driving system of claim 3, wherein at least two of the first magnetic generators are arranged on the rear surface of the bed such that the first coil tips face each other.

5. The magnetic driving system of claim 3, wherein the first coil tip has a protrusion with an end facing the bed.

6. The magnetic driving system of claim 3, wherein the first coil tip has a protrusion facing the bed and is inclined at a predetermined angle toward the bed.

7. The magnetic driving system of claim 3, wherein the first coil tip has a bent end to face the bed.

8. The magnetic driving system of claim 1 or 2, wherein the second magnetic generator includes a second magnetic coil around which a coil is wound and having second coil tips formed at both ends thereof.

9. The magnetic driving system of claim 8, wherein the second coil tip has a protrusion with both ends facing the bed.

10. The magnetic driving system of claim 8, wherein the second coil tip is bent at both ends to face the bed.

11. The magnetic driving system of claim 8, wherein the second magnetic generator is provided in plurality.

12. The magnetic driving system of claim 4, wherein at least two first magnetic generators are connected to each other by extending the magnetic yoke.

13. The magnetic driving system of claim 1 or 2, wherein the first magnetic generator and the second magnetic generator are connected to each other by a magnetic yoke.

14. The magnetic driving system of claim 3, wherein a cross-section of the first magnetic coil has a circular or square shape.

15. The magnetic driving system of claim 8, wherein a cross-section of the second magnetic coil has a circular or square shape.
